# EUROPEAN PATENT APPLICATION

(11) **EP 4 491 096 A1**
(43) Date of publication of application: **15.01.2025**
(21) Application number: 23184516.5
(22) Date of filing: 10.07.2023
(51) Int. Cl.: A61B 3/00, A61F 9/008

(54) **DEVICES AND METHODS FOR AUGMENTATION OF AN IMAGE OF AN EYE DURING A MEDICAL PROCEDURE**

(71) Applicant: Leica Microsystems NC, Inc., Durham, NC 27703 (US)
(72) Inventor: Maione, Bryan, Durham, 27703 (US)
(74) Representative: J A Kemp LLP

(57) **Abstract**

A first aspect of this disclosure is related to a device for augmentation of an image of an eye during a medical procedure,
configured to:
- obtain a first image of an eye ;
- obtain a first positional information related to the first image , wherein the first positional information is related to at least one of a state of the eye or a medical procedure to be performed at the eye;
- obtain a second image of the eye during the medical procedure;
- determine a second positional information based on the first image, on the first positional information and on the second image;
- provide the second positional information together with the second image to a user.

## Description

### Technical Field

This disclosure is related to devices and methods for augmentation of an image of an eye during a medical procedure. Furthermore, a microscope for augmentation of an image is disclosed.

### Background

Eye surgeries are medical procedures that involve the treatment of various eye conditions, including cataracts, glaucoma, refractive errors, and other vision-related issues. Intraocular lens (IOL) surgery is a common type of eye surgery that involves the replacement of the natural lens of the eye with an artificial lens. This procedure is typically performed to treat cataracts, which occur when the natural lens becomes cloudy, causing vision impairment.

Eye surgeries are sometimes supported by augmented reality information, which is normally shown to the surgeon on a separate monitor. Furthermore, pre-operative information is used to support the surgeon during the medical procedure. However, existing systems only provide general and/or static information. Improvements are desirable.

### Summary

An object of this disclosure is the improvement of an ophthalmologic medical procedure with augmented information.

This object is solved by the disclosed embodiments, which are defined in particular by the subject matter of the independent claims. The dependent claims provide further embodiments. Various aspects and embodiments of these aspects are also disclosed in the following summary and description, which provide additional features and advantages.

A first aspect of this disclosure is related to a device for augmentation of an image of an eye during a medical procedure,
configured to:
- obtain a first image of an eye ;
- obtain a first positional information related to the first image , wherein the first positional information is related to at least one of a state of the eye or a medical procedure to be performed at the eye;
- obtain a second image of the eye during the medical procedure;
- determine a second positional information based on the first image, on the first positional information and on the second image;
- provide the second positional information together with the second image to a user.

A device for augmentation of an image of an eye can be implemented in hardware and/or in software. In particular, such a device can be implemented only in software and configured to operate on a general-purpose computer and/or a specialized hardware.

A medical procedure can comprise a set of actions or steps taken by a medical professional to diagnose, treat, or prevent a medical condition. A medical procedure can range from simple and/or minimally invasive procedures, such as a physical examination, to more complex and invasive procedures, such as surgery. Medical procedures can involve the use of various medical tools and technologies, including diagnostic tests, imaging equipment, and/or surgical instruments. They may be performed in a variety of settings, including hospitals, clinics, and outpatient facilities. An example of a medical procedure can be a cataract surgery. Thereby, a clouded lens is removed from an eye, and replaced with an artificial lens implant, e.g. an IOL. Another example of a medical procedure is a glaucoma surgery. Thereby, an intraocular pressure inside the eye is reduced, e.g. by drainage implants.

A first image can be obtained before the medical surgery. For example, the first image can be taken during an examination procedure that is conducted in preparation of the medical procedure. Additionally or alternatively, a first image can be obtained during another medical procedure, in particular during another surgery. A device according to the first aspect can receive the first image from an external source. Additionally or alternatively, a device can fetch the first image from an external source, e.g., a shared memory. Furthermore, the device can generate the first image on its own. In this case, the device can comprise a camera. For example, the device can be a microscope with a camera.

Within the first image of the eye a biometric feature of the eye can be identified. A biometric feature can be, e.g., a part of an iris or a part of a pupil. Both organs have distinct patterns and structures that are unique to each individual. Furthermore, a biometric feature can be or comprise a part of one or more blood vessels, for example in the sclera.

A first positional information can relate to a medical condition of an eye, e.g. one or more axes of astigmatism. Additionally or alternatively, first positional information can relate to information where an examination and/or a medical treatment should be performed, e.g., an incision point, an incision trajectory and/or an IOL placement. A positional information can comprise a position, a distance, an area, a circumference, a location and/or an orientation. Additionally or alternatively, first positional information can also relate to a gradient, in particular with respect to position or time.

A second image, which is obtained during the medical operation, can be obtained with the same or with a different device than the first image. In particular, a second image can be obtained by a microscope, or a camera attached to a microscope. A second image (and also a first image) can capture a complete eye or only one or more parts of it. A device according to the first aspect can receive and/or fetch the second image from an external source. Furthermore, the device can generate the second image on its own. In this case, the device can comprise a camera. For example, the device can be a microscope with a camera.

A second positional information can comprise the first positional information which is aligned to the second image. For example an axis of astigmatism as identified as first positional information for the first image is transformed as second positional information to fit into the second image. This can be done by taking information of the first image, which is also present in the second image and determine a difference between the two images. Based on this difference, the second positional information is then determined as the first positional information that is transformed to the context of the second image. The determination of the difference can be difficult because the second image can differ from the first image in perspective, resolution, details, and/or is noisy, e.g. by a light of a microscope or a camera by which the second image is captured. Therefore, the information of the first image and the information of the second image that is used to determine the difference of the size and orientation of the eye within the two images can be manifold. For example, it can comprise positional or chromatic information of certain parts of the eye, e.g. of a blood vessel, an eye lid, and/or a structure in the pupil. Additionally or alternatively, an artificial scale present and standardized in the first and in the second image can be used to calculate the difference between the two images.

The second positional information together with the second image can be provided in such a format that it can be displayed as an augmented reality to a user. The second positional information can be provided as an augmented information within the second image. Additionally or alternatively, the second positional information can be provided separately with the second image such that both kinds of information can be displayed independently from each other. In an embodiment, the device can be configured to adapt the first image based on the second image; and to display the adapted first image together with the second image to a user. Advantageously, the second image can be augmented with the first image, e.g. by an overlay and/or by a picture-in-picture arrangement.

Advantageously, with a device according to the first aspect, an accurate augmentation of an image captured during a medical procedure is possible.

An embodiment of the first aspect is related to a device,
configured to:
- determine at least one of a scaling difference, a rotational difference, or a translational difference between the first image and the second image; and
- determine the second positional information on at least one of the identified differences.

A geometrical difference can be determined based on a visual comparison of the first and the second image. The visual comparison can be based on positional and/or chromatic information in the image. For example, the visual comparison can be based on a biometrical structure within the eye that can be identified in the first and in the second image. This can be, for example, the trajectory of an eye lid, or a blood vessel within the eye, or an invariant form in the pupil of the eye. Advantageously, an alignment of the first positional information based on a geometrical difference provides an effective augmentation of a second image.

An embodiment of the first aspect is related to a device,
configured to determine the second positional information based on a non-iterative difference identification.

A rough adaptation can provide a fast and computationally efficient determination of starting values for an optimization of the alignment for the second image.

An embodiment of the first aspect is related to a device,
configured to determine the second positional information based on:
- the seat position of the person that performs the medical procedure; and/or - the eye being a left eye or a right eye.

The seat position and or the position of the eye can in particularly be provided as a manual input by the surgeon or other personal. Thereby, advantageously, a more accurate alignment is possible.

An embodiment of the first aspect is related to a device,
configured to adapt the second positional information based on:
- a scale of the eye of the first image relative to the eye of the second image based on a biometrical feature of the eye;
- a rotation of the eye of the first image relative to the eye of the second image based on the eye being a left eye or a right eye;
- a translation between the eye of the first image relative to the eye of the second image based on a center of a pupil of the eye.

Scale-based adaptation can be performed, e.g., based on scale-dependent features of the first and the second image that can be identified as representing the same feature. Thereby, a key structure in the first and the second image can be identified, e.g. an eye lid. Based on the identified key structure the first image can be scaled such that it matches the second image. The method can also be applied for the matching of rotation and/ or translation of the first and the second image. Based on this a more accurate alignment is possible.

An embodiment of the first aspect is related to a device,
configured to determine the second positional information based on an iterative difference identification.

An iterative method can be used to refine an estimate of a geometrical difference between the first and the second image. An iterative method can comprise a deterministic and/or statistic optimization. An iterative method can comprise a gradient descent and/or a machine learning algorithm. Advantageously, based on an iterative method a precise alignment can be obtained.

An embodiment of the first aspect is related to a device,
configured to perform the iterative difference identification based on the non-iterative difference identification.

A non-iterative difference identification can provide the starting values for the iterative difference identification. In particular, a coarse but computationally efficient difference identification can be used to obtain one or more starting values for a computationally more complex but also more precise iterative alignment. In this way the complex iterative alignment will also be performed efficiently, since it only deals with one or more small differences in order to obtain second positional information.

An embodiment of the first aspect is related to a device,
configured to:
- obtain a third image during the medical procedure;
- determine a third positional information based on the second positional information and on the third image;
- provide the third positional information together with the third image to the user.

A third image can capture a complete eye or only one or more parts of it. By a third image further situational changes that take place during the medical procedure can be taken into account. Such changes can, e.g., relate to an incision and/or a lens placement. The third image can also be the next image in an image stream provided by the camera that also provided the second image. The third image can also be followed by further images. An alignment of positional information to the third image can be implemented in the same way as an alignment of positional information to a second image.

Advantageously, augmentation information can be aligned to each frame of a video stream during medical procedure. If the difference between the images during the medical procedure is small, a difference identification can be based on an accurate iterative alignment method. Thereby, an accurate alignment of positional information to an image can be provided during the medical procedure in real time.

An embodiment of the first aspect is related to a device,
configured to determine:
- the second positional information based on a non-iterative method and on an iterative method; and
- the third positional information based on an iterative method.

An embodiment of the first aspect is related to a device,
configured to determine at least one of the first, the second, or the third positional information by a neural network, in particular a convolutional neuronal network and/or a regression neuronal network.

Based on a neural network a precise alignment can be obtained, in particular the second and/or the third positional information can be computed highly accurately. A neural network can comprise a standard neural network, e.g. VGG and/or ResNET. VGG is a convolutional neural network architecture that is characterized by its use of small 3x3 convolutional filters and deep stacking of convolutional layers. ResNET is a deep convolutional neural network architecture that uses residual connections to enable training of very deep neural networks, up to hundreds of layers deep. In particular, a convolutional neural network can be used for efficient image processing. To determine continuous variables for scaling, translation, and rotation a fully connected single or multilayer regression neural network can be used. The regression neural network can be a feedforward neural network, such as a multilayer perceptron. The regression neuronal network in particular can be configured to obtain its input from a convolutional neural network. Thereby, a geometric difference can be computed with high accuracy.

An embodiment of the first aspect is related to a device,
wherein the neural network is configured to determine at least one of:
- a bounding box;
- an angle or an angle-related value.

A bounding box can be a rectangular frame that encloses a part of an eye and/or region of interest in an eye in an image. For example, a bounding box can enclose the lens and/or an incision trajectory. By identifying and localizing objects with bounding boxes, computer algorithms can be trained to recognize and classify objects in a supervised learning setting. A bounding box needs not to be a rectangle but can have another shape such as a square, a circle, a trapezoid, and/or a polygon. An angle or an angle-related value can be, e.g., a value in degrees, radians, or gradians. Furthermore, an angle related value can be a trigonometric value, e.g. a sine and/or cosine value.

An embodiment of the first aspect is related to a device,
configured to determine at least one of the first, the second, or the third positional information based on an optimization comprising at least one of:
- a per pixel loss function; or
- a perceptual loss function.

A per-pixel-loss-function measures the difference between two images, e.g. the first and the second image, by comparing the values of their individual pixels. In a per pixel loss function, the difference between the pixels in the two images is computed and then aggregated across all pixels to obtain a scalar value that represents the overall difference between the images. A per-pixel-loss-function can comprise the mean squared error and/or the mean absolute error.

A perceptual loss function is designed to measure the perceptual similarity between two images, e.g. between the first and the second image. A perceptual loss function can, e.g. comprise a measure for content loss. Content loss is based on feature representations. Feature representations capture high-level information about the content of images, such as edges, textures, and objects. An output of a convolutional neural network can be taken as a feature representation. The content loss function can measure a distance between the feature representations of two images, e.g. by using mean squared error. Additionally or alternatively, a perceptual loss function can comprise a style loss function, which measures a similarity of the texture and color patterns between two images.

An embodiment of the first aspect is related to a device,
wherein the neural network is at least partly manually trained.

Hand-labelled bounding boxes can be inserted into training images. A difference between estimated bounding boxes and hand-labelled bounding boxes can then be calculated and used as a loss value for training the network. This can in particular be based on an intersection-over-union loss function. Additionally and alternatively other parameters for aligning different images can be based on self-supervised learning, e.g. a rotation difference and/or a translation difference.

A second aspect of this disclosure is related to a method for augmentation of an image of an eye during a medical procedure,
with the steps:
- obtaining a first image of an eye , before a medical procedure;
- obtaining a first positional information related to the image, wherein the first positional information is related to at least one of a state of the eye or a medical procedure to be performed at the eye, before the medical procedure;
- obtaining a second image of the eye during the medical procedure;
- determining a second positional information based on the first image, on the first positional information and on the second image;
- providing the second positional information together with the second image to a user.

A third aspect of this disclosure is related to a microscope,
configured to:
- perform a method according to the second aspect; or
- to interact with a device according to one of the first aspect.

### Short Description of Figures

Further advantages and features result from the following embodiments, some of which refer to the figures. The figures do not always show the embodiments to scale. The dimensions of the various features may be enlarged or reduced accordingly, in particular for clarity of description. For this purpose it is shown, partially schematized, in:
Fig. 1 - an alignment of positional information of a first image to a second image, according to an embodiment of this disclosure;
Fig. 2 - a flow chart for generating of a second positional information within a second image, according to an embodiment of this disclosure;
Fig. 3 - two neuronal network systems for identifying a rotational shift and a pupil-area according to an embodiment of this disclosure;
Fig. 4 - a runtime graph for a feature identification in two consecutive images, according to an embodiment of this disclosure;
Fig. 5 - a microscope with an augmented image presentation, according to an embodiment of this disclosure.

In the following description reference is made to the accompanying figures which form part of the disclosure, and which illustrate specific aspects in which the present disclosure can be understood. Identical reference signs refer to identical or at least functionally or structurally similar features.

In general, a disclosure of a described method also applies to a corresponding device (or apparatus) for carrying out the method or a corresponding system comprising one or more devices and vice versa. For example, if a specific method step is described, a corresponding device may include a feature to perform the described method step, even if that feature is not explicitly described or represented in the figure. On the other hand, if, for example, a specific device is described on the basis of functional units, a corresponding method may include one or more steps to perform the described functionality, even if such steps are not explicitly described or represented in the figures. Similarly, a system can be provided with corresponding device features or with features to perform a particular method step. The features of the various exemplary aspects and embodiments described above or below may be combined unless expressly stated otherwise.

### Detailed Description of the Figures

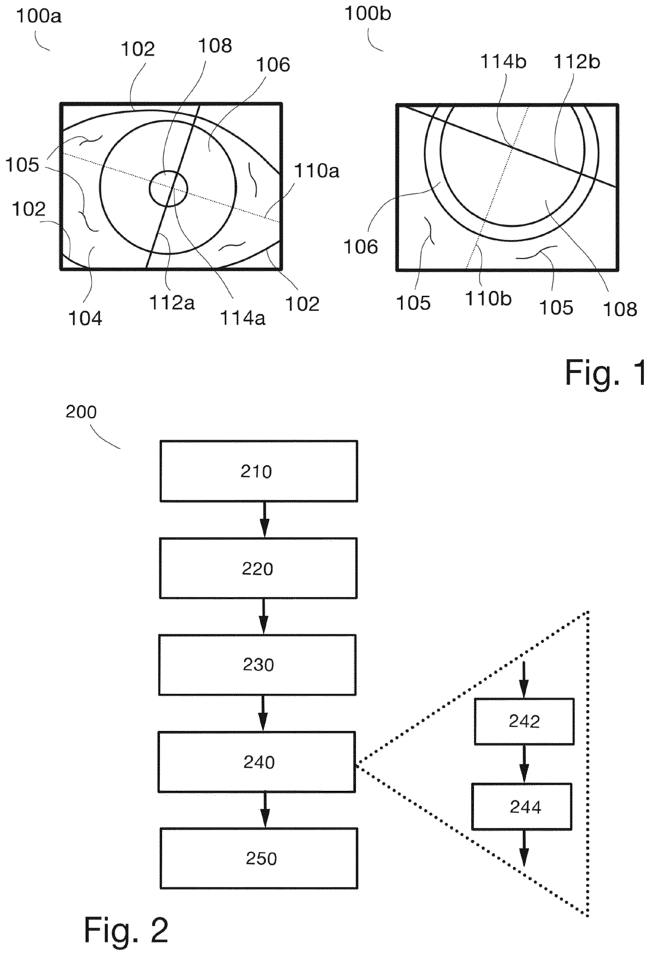

Fig. 1 illustrates an alignment of positional information of a first image 100a to a second image 100b as an output of a device according to the first aspect of this disclosure.

A first image 100a provides a perspective on an eye with lids 102, a sclera 104, blood vessels 105, an iris 106, and a pupil 108. Positional and chromatic information about the detected features of the eye are stored as first positional information. The first image 100a is captured during a pre-operative assessment of the patient. In this case, the patient suffers from cataract. During pre-operative scans two axes of astigmatism 110a, 112a have been identified and related to the first image 100a. This information is also stored as first positional information. Furthermore, the visual axis 114a, which connects the center of the region of highest acuity of the retina (fovea) with the center of curvature of the cornea, has also been identified during pre-operative scans and additionally assigned to the first positional information. The identified information will be important for a surgeon in a subsequent surgery. During the surgery, the natural deteriorated lens behind the pupil of the patient's eye will be removed and an artificial lens, IOL, will be implanted.

A second image 100b shows the eye from a slightly different perspective. In this perspective the eye lids 102 are not visible. Furthermore, the second image 100b is shifted about 90° relative to the first image 100b. Only the sclera 104, the iris 106 and the pupil 108 are visible. The second image 100b is captured at the beginning of the surgery.

Based on the positional and chromatic information of the blood vessels 105 a scaling difference, a rotational difference, and a translational difference between the first image and the second image are determined. (Additionally or alternatively, any feature common to both images can be used, e.g. structures in the iris, scleral pigmentations, etc.) Based on the determined difference information, the axes of astigmatism 110b, 112b, and the visual axis 114b are determined in the second image. As can be seen in the figure, the aligned axes of astigmatism 11 0b, 112b are shifted by about 90°. Furthermore, the parts of the eye 104, 106, and 108 are determined in the second image as well. As can be seen in the second image, the pupil 108 is larger in relation to the iris 106. Based on the determined alignment, the positional information, in particular the axis of astigmatism 110b, 112b, and the visual axis 114b can be provided as augmented information in the second image. Thereby, the surgeon receives important information for the success of the medical procedure.

Fig. 2 illustrates a flow chart of a method 200 for generating a second positional information within a second image, according to an embodiment of this disclosure.

In a first step 210 a first image of an eye is obtained, e.g. the first image 100a of Fig. 1. The first image can be passively received and/or actively fetched by the computing device that carries out the method. The first image can furthermore be obtained prior to a medical procedure, such as a surgery, during a pre-operative examination. The first image can be captured by arbitrary imagery devices.

In a second step 220 a first positional information related to the first image is obtained. The first positional information is related to at least one of a state of the eye and/or a medical procedure to be performed at the eye. Hence, the first positional information can comprise an axis of astigmatism, a circumference of the pupil and/or of the sclera, an indication of an area in the eye that is relevant for the medical procedure, a point and/or a trajectory of incision, or a location where an IOL should be located.

In a third step 230 a second image of the eye is captured. This step takes place during the medical procedure, e.g. by a microscope that is used in the medical procedure. The second image differs from the first image. Hence, the first positional information cannot be used within the context of the second image.

Therefore, a second positional information is determined in a fourth step 240. The second positional information is determined based on information provided by the first image and the second image. This information can be, e.g., a biometrical feature. If this features is present in the first image and the second image it can be taken to determine the geometric and chromatic differences between the first and the second image. Once the geometric and/or chromatic transformation between the first and the second image is determined, the second positional information is determined as the first positional information transformed into the context of the second image.

The determination of second positional information in step 240 of the method 200 can comprise a number of substeps. In an embodiment of the method 200, the alignment of the first positional information in the second image as second positional information is performed in two substeps.

In a first sub-step 242 a coarse alignment is performed. Thereby, first values for scale, a rotation, and a translation difference between the first image and the second image are defined. The scale difference is based on a size of the iris in both images. The iris size can be automatically detected. The rotation difference is determined based on a detection of the left eye and of the right eye (OD/OS) in both images. This information can be manually provided by medical personnel. The translation difference is determined based on an identified center of the detected pupil and/or based on a position of another biometric feature. This can also be automatically.

In a second sub-step 244 a fine alignment is performed. An iterative optimization is used for the fine alignment 244. A minimization of a perceptual loss is performed. A perceptual loss function is used in combination with a neural network for the image alignment. The perceptual loss function is configured to measure the perceptual similarity between two first and the second image. As an alternative to a perceptual loss function also a per-pixel loss function can be implemented.

After the second positional information has been determined, it is provided together with the second image for display to a user in a fifth step 250. Therefore, the second positional information is merged with the second image to provide an augmented second image. The augmented second image can be a single isolated image.

Additionally or alternatively, the augmented second image can be part of an image stream, i.e. a video stream. In this case, a third positional information is determined for a third image, which is a consecutive image to the second image. And the third positional information is determined based on the second image, the third image and the second positional information. This is done in the same way the second positional information is determined for the second image. In this way also further positional information for further images can be provided enabling an augmented video stream with continuously updated positional information about the state of the eye and/or about the medical procedure.

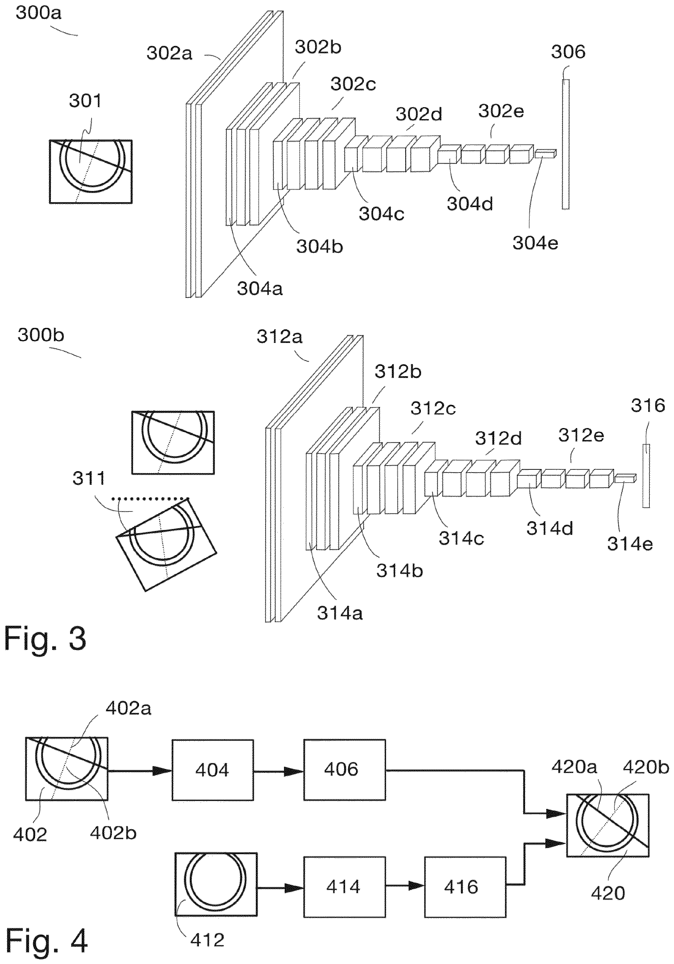

Fig. 3 illustrates a system with two neuronal networks as a part of an embodiment of a device according to the first aspect. A first neural network 300b is configured determine a center of a pupil. A second neural network 300a is configured for determining a rotational shift between two images, e.g. between a first image and a second image.

The first neural network 300a and the second neural network 300b are convolutional neuronal networks. They are trained on a perceptual loss function. The perceptual loss functions can be implemented using pre-trained convolutional neural network (CNN). The pre-trained CNN can be configured as feature extractor to extract high-level features from images.

The perceptual loss function typically consists of two main components: a content loss and a style loss. The content loss measures the difference between the high-level features of the first image and the second image, while the style loss measures the difference in the lower-level features, such as textures and colors, in both images. To implement a perceptual loss function, a pre-trained CNN, such as VGG-16 or ResNet, is chosen. Then features from the intermediate layers of the network are extracted. The content loss can then be computed by comparing the feature maps of the first image and the second image at a specific layer of the network. The style loss can be computed by comparing the correlations between the feature maps of the first image and the second image across multiple layers of the network. Once the content and style losses are computed, they are combined to form the overall perceptual loss function. This perceptual loss function can be used to train the neural network. As an alternative to a perceptual loss function a per-pixel loss function can be implemented.

The first neural network 300a and the second neural network 300b are based on the VGG16 standard architecture for convolutional neural networks. However, in contrast to the standard VGG16 architecture, the neural networks 300a, 300b comprise 13 convolutional layers and a single fully connected layer for a final regression analysis (The standard VGG16 comprises 13 convolutional layers and three fully connected layers for a classificator.).

In the following, the structure of the first neural network 300a is explained. An entry layer comprises two convolutional layers 302a with a size of 224x224x64. A first hidden layer comprises a max pooling layer 304a that samples the initial image down to a size of 112x112x128 by using a 3x3 receptive field. The first hidden layer furthermore comprises two convolutional layers 302b. A second hidden layer performs a downsampling to 56x56x256 by a max pooling layer 304b and is followed by three convolutional layers 302c. A third hidden layer performs a downsampling to 28x28x512 by a max pooling layer 304c and is followed by three convolutional layers 302d. A fourth hidden layer performs a downsampling to 14x14x512 by a max pooling layer 304d and is followed by three convolutional layers 302e. A final max pooling layer 304e performs a further downsampling to 7x7x512. This layer is connected to a single 8x1x1 fully connected layer 306 for a regression analysis.

The first neural network is trained with discrete frames of surgical video as input and corresponding bounding boxes for the iris and pupil for labels. Labels are gathered from human labeling. During training, the first neural network is optimized via supervised back-propagation to maximize the intersection over union of the predicted bounding boxes and human gathered labels for each surgical frame.

The operation of the first neural network is explained now. A first surgical image is aligned to the pre-operative imagery as per steps 242 and 244 of Fig. 2. Afterwards, based on the alignment to the pre-operative imagery, the first neural network 300a infers the center of the pupil 301 of the eye yielding information about a top of the pupil, a left edge of the pupil, a maximum height of the pupil, and a maximum width of the pupil. The first neural network can also be used to infer the iris of the eye in the same way. Therefore, the neural network can also be used in realtime on consecutive images during the medical procedure to provide a continuous centering and/or scaling of the image (in this case no further alignment is performed).

The second neural network 300b has a similar structure. It comprises 13 convolutional layers 312a, 312b, 312c, 312d, 312e that are transformed by 5 max pooling layers 314a, 314b, 314c, 314d, 314e. However, instead of an 8x1x1 fully connected layer, a 2x1x1 fully connected layer 316 for a regression analysis is used for the second neural network 300b.

The second neural network is trained with discrete frames of surgical video as well. In contrast to the first neural network, the second is trained using the semi-supervised method as follows. A copy of each surgical frame is produced, the copy is rotated by a randomly determined angle θ 311. The original non-rotated image, as well as the copied rotated image are provided to the network as inputs. The network is then optimized via back-propagation to minimize the mean difference between the predicted angle θ, and its true value. In this way, no human labels are required.

The operation of the second neural network 300b is explained now. A centered image, i.e. an image corrected by the parameters obtained from the first neural network 300a is used for an inference of translational shift and scale between two images. Afterwards, based on the alignment of translation and scale, the second neural network 300b infers an angle θ or a trigonometric value (cos(θ), sin(θ)) of the image or of the eye in the image. Therefore, the neural network can also be used in realtime on consecutive images during a medical procedure to provide a continuous rotational alignment of the image and/or of the eye (in this case no further alignment is performed).

Fig. 4 provides a runtime graph for a feature identification in two consecutive images, according to an embodiment of this disclosure. The graph is exemplary for the realtime alignment explained in connection with Fig. 3. A first image 402 is provided to the machine learning algorithm. The first image comprises first positional information 402a, 402b which relates to two axes of astigmatism. In a first step 404, a center of the pupil (or the iris) depicted in the first image 402 is predicted. This can be done, e.g., by the convolutional neural network 300a of Fig. 3. As a result an information about the center of the eye is obtained in step 406.

Concurrent to this process, a second image 412, which was captured after the first image, also undergoes a center detection 414 resulting in a centered image 416. Based on the centered first and second image an angle difference is inferred, e.g. by the neural network 300b. Once the angle is inferred the axes of astigmatism 420a, 420b can be related, as second positional information, to the second image to obtain an augmented second image 420.

In general for this method, the prediction for each frame is relative to the previous frame. The obtained information can be propagated all the way back to the initial image, e.g. the image that was used for pre-operative registration. The method can be used for augmenting a video stream during a medical surgery and provide a surgeon updated important information related to the status of the eye or the medical procedure performed.

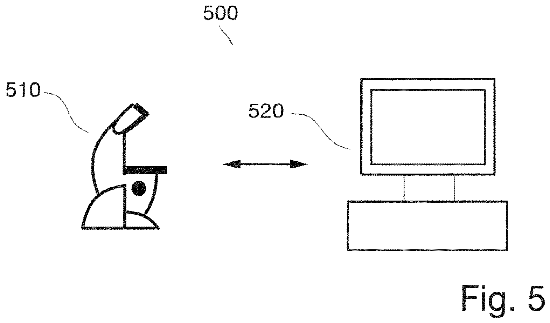

Some embodiments relate to a microscope comprising a system as described in connection with one or more of the Figs. 1 to 4. Alternatively, a microscope may be part of or connected to a system as described in connection with one or more of the Figs. 1 to 4. Fig. 5 shows a schematic illustration of a system 500 configured to perform a method described herein. The system 500 comprises a microscope 510 and a computer system 520. The microscope 510 is configured to take images and is connected to the computer system 520. The computer system 520 is configured to execute at least a part of a method described herein. The computer system 520 may be configured to execute a machine learning algorithm. The computer system 520 and microscope 510 may be separate entities but can also be integrated together in one common housing. The computer system 520 may be part of a central processing system of the microscope 510 and/or the computer system 520 may be part of a subcomponent of the microscope 510, such as a sensor, an actor, a camera or an illumination unit, etc. of the microscope 510.

The computer system 520 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 520 may comprise any circuit or combination of circuits. In one embodiment, the computer system 520 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 520 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 520 may include one or more storage devices, which may include one or more memory elements suitable to the particular application, such as a main memory in the form of random-access memory (RAM), one or more hard drives, and/or one or more drives that handle removable media such as compact disks (CD), flash memory cards, digital video disk (DVD), and the like. The computer system 520 may also include a display device, one or more speakers, and a keyboard and/or controller, which can include a mouse, trackball, touch screen, voice-recognition device, or any other device that permits a system user to input information into and receive information from the computer system 520.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine-readable carrier. Other embodiments comprise the computer program for performing one of the methods described herein, stored on a machine-readable carrier. In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Embodiments are based on using a machine-learning model or machine-learning algorithm. Machine learning may refer to algorithms and statistical models that computer systems may use to perform a specific task without using explicit instructions, instead relying on models and inference. For example, in machine-learning, instead of a rule-based transformation of data, a transformation of data may be used, that is inferred from an analysis of historical and/or training data. For example, the content of images may be analyzed using a machine-learning model or using a machine-learning algorithm. In order for the machine-learning model to analyze the content of an image, the machine-learning model may be trained using training images as input and training content information as output. By training the machine-learning model with a large number of training images and/or training sequences (e.g. words or sentences) and associated training content information (e.g. labels or annotations), the machine-learning model "learns" to recognize the content of the images, so the content of images that are not included in the training data can be recognized using the machine-learning model. The same principle may be used for other kinds of sensor data as well: By training a machine-learning model using training sensor data and a desired output, the machine-learning model "learns" a transformation between the sensor data and the output, which can be used to provide an output based on non-training sensor data provided to the machine-learning model. The provided data (e.g. sensor data, meta data and/or image data) may be preprocessed to obtain a feature vector, which is used as input to the machine-learning model.

Machine-learning models may be trained using training input data. The examples specified above use a training method called "supervised learning". In supervised learning, the machine-learning model is trained using a plurality of training samples, wherein each sample may comprise a plurality of input data values, and a plurality of desired output values, i.e. each training sample is associated with a desired output value. By specifying both training samples and desired output values, the machine-learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g. a classification algorithm, a regression algorithm or a similarity learning algorithm. Classification algorithms may be used when the outputs are restricted to a limited set of values (categorical variables), i.e. the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are. Apart from supervised or semi-supervised learning, unsupervised learning may be used to train the machine-learning model. In unsupervised learning, (only) input data might be supplied, and an unsupervised learning algorithm may be used to find structure in the input data (e.g. by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Furthermore, some techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e. outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g. a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may e.g. be used to store, manipulate or apply the knowledge.

Machine-learning algorithms are usually based on a machine-learning model. In other words, the term "machine-learning algorithm" may denote a set of instructions that may be used to create, train or use a machine-learning model. The term "machine-learning model" may denote a data structure and/or set of rules that represents the learned knowledge (e.g. based on the training performed by the machine-learning algorithm). In embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g. of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e. to achieve a desired output for a given input.

Alternatively, the machine-learning model may be a support vector machine, a random forest model or a gradient boosting model. Support vector machines (i.e. support vector networks) are supervised learning models with associated learning algorithms that may be used to analyze data (e.g. in classification or regression analysis). Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection.

### List of Reference Signs

- 100a: first image
- 100b: second image
- 102: eye lids
- 104: sclera
- 105: blood vessels
- 106: iris
- 108: pupil
- 110a: axis of astigmatism
- 110b: aligned axis of astigmatism
- 112a: axis of astigmatism
- 112b: aligned axis of astigmatism
- 114a: visual axis
- 114b: aligned visual axis
- 200: method
- 210: obtaining first image
- 220: obtaining first positional information
- 230: obtaining second image
- 240: determining second positional information
- 242: coarse alignment
- 244: fine alignment
- 250: display of second positional information, second image
- 300a: CNN for angle difference detection
- 300b: CNN for center detection
- 301: size of pupil
- 302a: convolutional layers of entry layer
- 302b: convolutional layers of first hidden layer
- 302c: convolutional layers of second hidden layer
- 302d: convolutional layers of third hidden layer
- 302e: convolutional layers of third hidden layer
- 304a: max pooling layer of first hidden layer
- 304b: max pooling layer of second hidden layer
- 304c: max pooling layer of second hidden layer
- 304d: max pooling layer of second hidden layer
- 304e: final max pooling layer
- 306: fully connected layer
- 311: rotational difference
- 312a: convolutional layers
- 312b: convolutional layers
- 312c: convolutional layers
- 312d: convolutional layers
- 312e: convolutional layers
- 314a: max pooling layer
- 314b: max pooling layer
- 314c: max pooling layer
- 314d: max pooling layer
- 314e: max pooling layer
- 316: fully connected layer
- 402: first image
- 402a: first positional information
- 402b: first positional information
- 404: iris detection
- 406: center detection
- 412: second image
- 414: center detection
- 416: centered image
- 420: augmented second image
- 420a: second positional information
- 420b: second positional information
- 500: microscope system
- 510: microscope
- 520: computer system

## Claims

1. A device for augmentation of an image of an eye during a medical procedure,
configured to:
- obtain a first image (1 00a) of an eye (102, 106, 108);
- obtain a first positional information (110a, 112a, 114a) related to the first image (100a), wherein the first positional information is related to at least one of a state of the eye or a medical procedure to be performed at the eye;
- obtain a second image (100b) of the eye (106, 108) during the medical procedure;
- determine a second positional information (110b, 112b, 114b) based on the first image, on the first positional information and on the second image;
- provide the second positional information together with the second image to a user.

2. The device according to the preceding claim,
configured to:
- determine at least one of a scaling difference, a rotational difference, or a translational difference between the first image and the second image; and
- determine the second positional information (110b, 112b, 114b) on at least one of the identified differences.

3. The device according to one of the preceding claims,
configured to determine the second positional information (110b, 112b, 114b) based on a non-iterative difference identification.

4. The device according to one of the preceding claims,
configured to determine the second positional information (110b, 112b, 114b) based on:
- the seat position of the person that performs the medical procedure; and/or
- the eye being a left eye or a right eye.

5. The device according to one of the preceding claims,
configured to adapt the second positional information (110b, 112b, 114b) based on:
- a scale of the eye of the first image relative to the eye of the second image based on a biometrical feature of the eye;
- a rotation of the eye of the first image relative to the eye of the second image based on the eye being a left eye or a right eye;
- a translation between the eye of the first image relative to the eye of the second image based on a center of a pupil of the eye.

6. The device according to one of the preceding claims,
configured to determine the second positional information based on an iterative difference identification (244).

7. The device according to one of the preceding claims,
configured to perform the iterative difference (244) identification based on the non-iterative difference identification (242).

8. The device according to one of the preceding claims,
configured to:
- obtain a third image (412) during the medical procedure;
- determine a third positional information (420a, 420b) based on the second positional information (402a, 402b) and on the third image;
- provide the third positional information together with the third image to the user.

9. The device according to the preceding claim,
configured to determine:
- the second positional information (110b, 112b, 114b) based on a non-iterative method and on an iterative method; and
- the third positional information based on an iterative method.

10. The device according to one of the preceding two claims,
configured to determine at least one of the first, the second, or the third positional information by a neural network, in particular a convolutional neuronal network and/or a regression neuronal network.

11. The device according to one of the preceding three claims,
wherein the neural network is configured to determine at least one of:
- a bounding box;
- an angle or an angle-related value (311).

12. The device according to one of the preceding four claims,
configured to determine at least one of the first, the second, or the third positional information based on an optimization comprising at least one of:
- a per pixel loss function; or
- a perceptual loss function.

13. The device according to one of the preceding five claims,
wherein the neural network is at least partly manually trained.

14. A computer-implemented method for augmentation of an image of an eye during a medical procedure,
with the steps:
- obtaining a first image (100a) of an eye (102, 106, 108), before a medical procedure;
- obtaining a first positional information (110a, 112a, 114a) related to the image, wherein the first positional information is related to at least one of a state of the eye or a medical procedure to be performed at the eye, before the medical procedure;
- obtaining a second image (100b) of the eye during the medical procedure;
- determining a second positional information (110b, 112b, 114b) based on the first image, on the first positional information and on the second image;
- providing the second positional information together with the second image to a user.

15. A microscope,
configured to:
- perform a method according to the preceding claim; or
- to interact with a device according to one of the preceding claims.
